# EUROPÄISCHE PATENTSCHRIFT

(11) **EP 1 931 616 B1**
(45) Veröffentlichungstag und Bekanntmachung des Hinweises auf die Patenterteilung: **29.01.2014**
(21) Anmeldenummer: 06793091.7
(22) Anmeldetag: 31.08.2006
(51) Int. Cl.: C07C 51/41, C07C 57/04, B01J 14/00, C08F 20/06, C08F 220/06

(54) **NEUTRALISATIONSVERFAHREN**
NEUTRALIZATION METHOD
PROCEDE DE NEUTRALISATION

(30) Priorität: 07.09.2005 DE 102005042604
(43) Veröffentlichungstag der Anmeldung: 18.06.2008
(60) Teilanmeldung: 10173421.8
(73) Patentinhaber: BASF SE, 67056 Ludwigshafen (DE)
(72) Erfinder: WEISMANTEL, Matthias, 63637 Jossgrund (DE); DE MARCO, Michael, 69469 Weinheim (DE); VAN ESBROECK, Dominicus, NL-4725 SN Wouse Plantage (NL); POSSEMIERS, Karl, J., B-2900 Schoten (BE); DE KAEY, Ronny, B-2531 Vremde (BE)
(86) Internationale Anmeldenummer: PCT/EP2006/065849
(87) Internationale Veröffentlichungsnummer: WO 2007/028751

(56) Entgegenhaltungen:
- EP-A- 1 470 905
- EP-A2- 0 372 706
- WO-A-03/095410
- DE-A1- 3 432 082

## Beschreibung

Die vorliegende Erfindung betrifft ein kontinuierliches Neutralisationsverfahren für ethylenisch ungesättigte Carbonsäuren sowie eine Vorrichtung zur Durchführung des Verfahrens.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ultmann's Encyclopedia of Industrial Chemistry, 6. Auflage, Band 35, Seiten 73 bis 103, beschrieben. Das bevorzugte Herstellungsverfahren ist die Lösungs- oder Gelpolymerisation. Bei dieser Technologie wird zunächst eine Monomermischung hergestellt, die diskontinuierlich neutralisiert und dann in einen Polymerisationsreaktor überführt wird, oder bereits im Polymerisationsreaktor vorgelegt wird. Im sich anschließenden diskontinuierlichen oder kontinuierlichen Verfahren erfolgt die Reaktion zum Polymergel, das im Falle einer gerührten Polymerisation bereits zerkleinert wird. Das Polymergel wird anschließend getrocknet, gemahlen und gesiebt und dann zur weiteren Oberflächenbehandlung transferiert.

Ein kontinuierliches Polymerisationsverfahren liegt beispielsweise der WO 01/38402 zugrunde, wobei die wässrige Monomerlösung kontinuierlich einem Mischkneter mit mindestens zwei achsparallel rotierenden Wellen zugeführt wird.

Kontinuierliche Gelpolymerisationen sind weiterhin bekannt aus WO 03/004237, WO 03/022896 und WO 01/016197.

Sowohl bei der kontinuierlichen als auch bei der diskontinuierlichen Polymerisation wird die Acrylsäure im Falle der Vorneutralisation diskontinuierlich neutralisiert. Typischerweise werden im Polymerisationsreaktor im Falle der Lösungspolymerisation die Edukte Acrylsäure, Wasser, optionale Comonomere und Natronlauge diskontinuierlich dosiert und vermischt. In diesem Schritt wird weitestgehend der restliche Polymerisationsverlauf und auch die zu erwartenden Polymereigenschaften festgelegt. Der Vernetzungsgrad des Basispolymers sowie der Neutralisationsgrad werden typischerweise in diesem Schritt bestimmt. Der Neutralisationsgrad der Monomeren liegt zwischen 0 und 80 mol-%. Im Falle der sauren Polymerisation wird das erhaltene Polymergel üblicherweise zu 50 bis 80 mol-%, vorzugsweise zu 60 bis 75 mol-% nachneutralisiert, indem Natriumhydroxid- oder Natriumcarbonatlösung zum sauren Polymergel zugegeben und eingearbeitet wird.

Neutralisationsverfahren werden beispielsweise in EP-A 0 372 706, EP-A 0 574 260, WO 03/051415 und EP-A 1 470 905 beschrieben.

EP-A 0 372 706 beschreibt ein dreistufiges Neutralisationsverfahren, bei dem in einer ersten Stufe Acrylsäure und Natronlauge gleichzeitig dosiert werden, so dass ein Neutralisationsgrad von 75 bis 100 mol-% eingehalten wird, in einer zweiten Stufe der Neutralisationsgrad auf 100,1 bis 110 mol-% angehoben wird um in der eingesetzten Acrylsäure als Verunreinigung enthaltene Diacrylsäure zu hydrolysieren, und in einer dritten Stufe durch Zusatz von weiterer Acrylsäure ein Neutralisationsgrad von 20 bis 100 mol-% eingestellt wird.

EP-A 0 574 260 offenbart auf Seite 7, Zeilen 38 bis 41, dass bei der Neutralisation vorteilhaft Natronlauge vorgelegt und anschließend Acrylsäure unter Kühlung zugesetzt wird.

WO 03/051415 lehrt ein Verfahren zur Herstellung wasserabsorbierender Polymere, bei dem die Monomerlösung eine Mindesttemperatur von 40°C aufweist.

EP-A 1 470 905 beschreibt in den Beispielen die kontinuierliche Neutralisation von Acrylsäure unmittelbar vor dem Polymeriationsreaktor. Aufgrund der Neutralisationswärme steigt die Temperatur auf 95°C.

WO 03/095410 A1 offenbart ein Neutralisationsverfahren, wobei Acrylsäuredämpfe am Kopf einer Rektifikationskolonne ohne Zwischenkondensation in Natronlauge absorbiert werden.

Es ist bekannt, dass sich die Reaktivität der Acrylsäure sehr stark von der ihrer Salze unterscheidet, weshalb auch der Polymerisationsverlauf stark abhängig ist vom pH-Wert, bei dem er stattfindet. In einer anschaulichen Darstellung in der Monographie "Modern Superabsorbent Polymer Technology", F. L. Buchholz und A.T. Graham, Wiley-VCH, 1998, oder in Ullmann's Encyclopedia of Industrial Chemistry, 6. Auflage, Band 35, Seite 35, ist die Polymerisationsgeschwindigkeit als Funktion des pH-Wertes aufgetragen. Demnach durchläuft die Polymerisationsgeschwindigkeit ein Minimum bei einem pH-Wert von 6 bis 7. Dies entspricht allerdings dem pH-Wert, der in aller Regel bei den Verkaufsprodukten gewünscht ist. Erklärt wird dieses Verhalten dadurch, dass es zwischen dem geladenen Monomeren in Salzform und der wachsenden Radikalkette zu elektrostatischen Abstoßungsreaktionen kommt, die im Falle der weitestgehend undissoziierten Acrylsäure nicht vorliegen, was zur Verlangsamung des Reaktionsyerlaufs führt.

Es ist auch bekannt, dass unneutralisierte Acrylsäure leichter polymerisiert werden kann als vorneutralisierte Systeme. Diese Differenz wird jedoch bei steigender Monomerkonzentration verringert, vor allem deswegen, weil eine höhere Monomerkonzentration die Dissoziation der Acrylsäuresalze unterdrückt.

Um all diesen reaktionstechnischen Details Rechnung zu tragen, werden bei der Reaktionsführung üblicherweise Kompromisse eingegangen.

Generell wird der Neutralisationsgrad der Acrylsäure bereits vor dem Eintritt in die kontinuierliche Polymerisation eingestellt. Die Neutralisation erfolgt diskontinuierlich. Die diskontinuierliche Neutralisation hat den Vorteil, dass die Dosierung von Acrylsäure und/oder Natronlauge temperaturgeregelt erfolgen kann. Damit werden Überhitzungen und unerwünschte Polymerisationen in dem Ansatzbehälter vermieden. Der Neutralisationsgrad wird entsprechend den Polymerisationsbedingungen und dem erwünschten Absorptionsprofil gewählt und wahlweise in einer nachgeschalteten Neutralisation, die zumeist am Polymergel erfolgt, korrigiert.

Nachteil der Neutralisation als vorgeschalteter diskontinuierlicher Verfahrensschritt ist vor allem der logistische Aufwand. Die Bereitstellung von Vorratstanks, Kesseln oder Behältnissen, welche die teilneutralisierte Acrylsäure bevorraten, sowie der komplette apparative Aufwand wirken sich nachteilig auf die Wirtschaftlichkeit des Prozesses aus.

Erwünschenswert wäre es daher, die Neutralisation kontinuierlich durchzuführen. Die kontinuierliche Neutralisation könnte dann vorteilhaft mit einer kontinuierlichen Polymerisation kombiniert werden, wobei der logistische Aufwand und die Bereitstellung von Vorratsbehältern minimiert wird. Die kontinuierliche Neutralisation erweist sich jedoch aufgrund der auftretenden Wärmeentwicklung als problematisch, da es dadurch zur unerwünschten vorzeitigen Polymerisation kommt. Der weitere Verlauf der kontinuierlichen Polymerisation wird durch bereits entstandenes Polymergel in allen Fällen negativ beeinträchtigt, insbesondere durch Verstopfungen in Leitungen, die nur für den Flüssigkeitstranspört ausgelegt wurden.

Aufgabe der vorliegenden Erfindung war die Bereitstellung eines kontinuierlichen Neutralisationsverfahrens, wobei das Auftreten unerwünscht hoher Temperaturspitzen vermieden werden kann.

Gelöst wurde die Aufgabe durch ein Neutralisationsverfahren, wobei mindestens eine ethylenisch ungesättigte Carbonsäure zumindest teilweise mit einer Base neutralisiert wird, dadurch gekennzeichnet, dass die Neutralisation kontinuierlich durchgeführt wird, die Temperatur der ethylenisch ungesättigten Carbonsäure von 0 bis 40°C beträgt, die neutralisierte Lösung gekühlt und teilweise in die Neutralisation rückgeführt wird, die rückgeführte neutralisierte Lösung in der Neutralisation nacheinander mit einer Base und ethylenisch ungesättigter Carbonsäure versetzt wird und die Temperatur der neutralisierten Lösung weniger als 70°C beträgt.

Vorzugsweise werden ethylenisch ungesättigte Carbonsäuren, wie Acrylsäure, Methacrylsäure, Maleinsäure, Fumarsäure und Itaconsäure, eingesetzt. Acrylsäure ist besonders bevorzugt.

Die Temperatur der ethylenisch ungesättigte Carbonsäure beträgt von 0 bis 40°C, vorzugsweise von 5 bis 35°C, besonders bevorzugt von 10 bis 30°C, ganz besonders bevorzugt von 15 bis 25°C, wobei auf einen ausreichenden Abstand zu Schmelzpunkt zu achten ist. Bei Verwendung von Acrylsäure sollte eine Temperatur von 15°C auf keinen Fall unterschritten werden.

Als Base ist wässriges Alkali bevorzugt. Wässriges Alkali sind alle alkalisch reagierenden wässrigen Lösungen, d.h. wässrige Lösungen mit einem pH-Wert von mindestens 8, vorzugsweise mindestens 10, besonders bevorzugt mindestens 12, ganz besonders bevorzugt mindestens 14.

Die im wässrigen Neutralisationsmittel einsetzbaren alkalischen Salze sind vorzugsweise Alkalimetallhydroxide, Alkalimetalloxide, Alkalimetallcarbonate und Alkalimetallhydrogencarbonate sowie deren Mischungen. Statt Alkalimetallsalzen können auch Ammoniumsalze verwendet werden. Natrium und Kalium als Alkalimetalle sind besonders bevorzugt, ganz besonders bevorzugt jedoch Natriumhydroxid, Natriumcarbonat oder Natriumhydrogencarbonat sowie deren Mischungen. Üblicherweise beträgt der Alkaligehalt im wässrigen Alkali mindestens 10 Gew.-%, vorzugsweise mindestens 20 Gew.-%, bevorzugt mindestens 30 Gew.-%, ganz besonders bevorzugt mindestens 40 Gew.-%.

Die Temperatur des wässrigen Alkali beträgt üblicherweise von 0 bis 45°C, vorzugsweise von 5 bis 40°C, besonders bevorzugt von 10 bis 35°C, ganz besonders bevorzugt von 15 bis 30°C, wobei Übersättigungen und damit Ausfällungen zu vermeiden sind.

Beträgt der Alkaligehalt des wässrigen Alkali mindestens 25 Gew.-%, so sind höhere Temperaturen vorteilhaft, üblicherweise von 10 bis 60°C, vorzugsweise von 20 bis 55°C, besonders bevorzugt von 30 bis 50°C, ganz besonders bevorzugt von 40 bis 45°C.

Das Verhältnis von ethylenisch ungesättigter Carbonsäure zu Base wird üblicherweise so gewählt, dass der Neutralisationsgrad der ethylenisch ungesättigter Carbonsäure nach der Neutralisation vorzugsweise von 25 bis 85 mol-%, bevorzugt von 27 bis 80 mol-%, besonders bevorzugt von 27 bis 30 mol-% oder 40 bis 75 mol-%, beträgt.

Der Neutralisationsgrad ist das Molverhältnis von neutralisierter ethylenisch ungesättigter Carbonsäure nach der Neutralisation zur Gesamtmenge eingesetzter ethylenisch ungesättigter Carbbonsäure vor der Neutralisation.

Die Neutralisation wird kontinuierlich durchgeführt. Dies bedeutet, dass dem Neutralisationsbereich ethylenisch ungesättigte Carbonsäure und/oder Base zugeführt wird und dem Neutralisationsbereich gleichzeitig neutralisierte Lösung entnommen wird. An- und Abfahrvorgänge des kontinuierlichen Neutralisationsverfahrens sind hiervon selbstverständlich ausgenommen.

Vorzugsweise wird die dem Neutralisationsbereich kontinuierlich entnommene Lösung zumindest teilweise kontinuierlich in die Polymerisation überführt.

Die Polymerisation wird vorzugsweise ebenfalls kontinuierlich durchgeführt.

Der Neutralisationsbereich ist der Bereich, in dem die Neutralisation weitgehend stattfindet, d.h. der Bereich in dem ethylenisch ungesättigte Säure und Base unter Salzbildung reagieren (Neutralisation).

Die Neutralisation ist weitgehend abgeschlossen, wenn der Umsatz der Neutralisation mindestens 90 mol-%, vorzugsweise mindestens 95 mol-%, bevorzugt mindestens 98 mol-%, ganz besonders bevorzugt mindestens 99 mol-%, beträgt. Der Umsatz kann leicht über die freigesetzte Neutralisationswärme durch Vergleich mit der theoretischen Wärmetönung ermittelt werden.

Die kontinuierliche Neutralisation wird so durchgeführt, dass die Temperatur der neutralisierten Lösung vorzugsweise weniger als 60°C, bevorzugt weniger als 50°C, besonders bevorzugt weniger als 40°C, ganz besonders bevorzugt weniger als 30°C, beträgt, wobei die Temperatur die Durchschnittstemperatur nach der Neutralisation ist, d.h. die Mitteltemperatur nach vollständiger Wärmetönung.

Mit steigender Temperatur steigt die Polymerisationsneigung der Lösung.

Der Abstand zwischen Neutralisation und Polymerisation beträgt üblicherweise mindestens 1 m, vorzugsweise mindestens 5 m, besonders bevorzugt mindestens 10 m, ganz besonders bevorzugt mindestens 20 m, und üblicherweise nicht mehr als 100 m, wobei der Abstand die Länge der Strecke ist, die die Monomerlösung auf geradem Weg zwischen der Alkalidosierung und Polymerisationsreaktor durchläuft.

Zusätzlich kann die neutralisierte Lösung mit Wasser verdünnt werden. Über die Verdünnung mit Wasser kann der Feststoffgehalt der neutralisierten Lösung eingestellt werden. Der Feststoffgehalt ist die Summe der Gewichtanteile an neutralisierter ethylenisch ungesättigter Carbonsäure und wahlweise überschüssiger ethylenisch ungesättigter Carbonsäure oder überschüssiger Base. Der Feststoffgehalt der neutralisierten Lösung beträgt typischerweise von 10 bis 80 Gew.-%, vorzugsweise von 20 bis 70 Gew.-%, besonders bevorzugt von 30 bis 60 Gew.-%.

Die Temperatur des Wassers beträgt üblicherweise von über 0 bis 40°C, vorzugsweise von 5 bis 35°C, besonders bevorzugt von 10 bis 30°C, ganz besonders bevorzugt von 15 bis 25°C.

Die neutralisierte Lösung wird gekühlt, wobei die zur Kühlung einsetzbaren Wärmeaustauscher keiner Beschränkung unterliegen. Die neutralisierte Lösung wird auf eine Temperatur von vorzugsweise weniger als 50°C, bevorzugt weniger als 40°C, besonders bevorzugt weniger als 30°C, ganz besonders bevorzugt weniger als 20°C, gekühlt. Die Kühlung sollte möglichst nah an der Neutralisation sein, da so hohe Verweilzeiten der neutralisierten Lösung bei hohen Temperaturen vermieden werden können.

Vorzugsweise werden Wasser und Base vorgemischt. In diesem Fall kann die dabei freiwerdende Lösungswärme bereits vor der Neutralisation, beispielsweise mittels geeigneter Wärmeaustauscher, abgeführt werden.

Ein Teil der neutralisierten Lösung wird in die Neutralisation rückgeführt.

Durch die Rückführung kann die Neutralisationswärme und die Lösungswärme besser verteilt und Temperaturspitzen (Peaktemperatur) in der Mischung niedrig gehalten werden. Der Anteil rückgeführter neutralisierter Lösung beträgt üblicherweise von 25 bis 99%, vorzugsweise von 33 bis 98%, besonders bevorzugt von 50 bis 95%, ganz besonders bevorzugt von 80 bis 90%, jeweils bezogen auf die neutralisierte Lösung.

Die ethylenisch ungesättigte Carbonsäure, die Base und wahlweise das Wasser können an beliebigen Stellen in die rückgeführte neutralisierte Lösung dosiert werden. Base und ethylenisch ungesättigte Carbonsäure werden nacheinander dosiert, ganz besonders bevorzugt Wasser, Base und ethylenisch ungesättigte Carbonsäure nacheinander.

Vorteilhaft wird mindestens eines der Edukte über zwei oder mehr getrennte Zugabestellen dosiert.

Beispielsweise können die Edukte über zwei, drei, vier, fünf oder sechs Zugabestellen dosiert werden, wobei die Zugabestellen vorzugsweise so angeordnet sind, dass sie eine gemeinsame Achse aufweisen (für zwei Zugabestellen) oder einen symmetrischen Stern bilden (für mindestens drei Zugabestellen) und die Achse bzw. Stern senkrecht zur Flussrichtung der neutralisierten Lösung befindet (Mehrfachzugabestellen).

Besonders vorteilhaft wird die Base dosiert, wenn zwei, drei oder vier Mehrfachzugabestellen hintereinander angeordnet werden.

Das Aufteilen in mehrere Zugabestellen bewirkt eine gleichmäßigere Durchmischung und niedrigere Temperaturspitzen, was die Gefahr unerwünschter Polymerisation vermindert.

In einer weiteren Ausführungsform werden Wasser und Base so dosiert, dass das Wasser die Base beim Eintritt in die Neutralisation umhüllt. Dazu können beispielsweise zwei ineinander gesteckte Rohre verwendet werden, wobei die Base über das Innenrohr und das Wasser über den Ringspalt zwischen innerem und äußeren Rohr dosiert werden.

Vorteilhaft enthält die Neutralisation einen zusätzlichen Behälter als Puffergefäß.

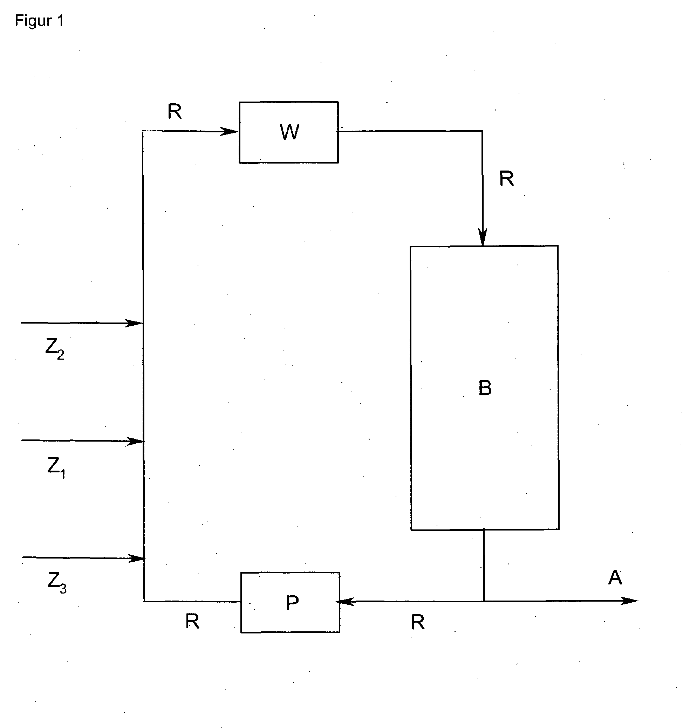

Eine beispielhafte erfindungsgemäße Neutralisation zeigt Figur 1, wobei die Bezugszeichen folgende Bedeutungen haben:
- Z₁ bis Z₃: Zuführungen für Edukte 1 bis 3
- A: Ableitung
- B: Behälter
- P: Pumpe
- R: Ringleitung
- W: Wärmeaustauscher

Mittels einer Pumpe P wird neutralisierte Lösung teilweise über die Ringleitung R rückgeführt. Der Rest der neutralisierten Lösung wird über die Ableitung A der weiteren Verwendung zugeführt. Der Behälter B dient als Puffer. Über die Zuleitung Z₁ wird vorzugsweise 50gew.-%ige Natronlauge, über die Zuleitung Z₂ wird vorzugsweise Acrylsäure und über die Zuleitung Z₃ wird vorzugsweise Wasser dosiert.

Damit die Edukte möglichst intensiv in die rückgeführte neutralisierte Lösung eingemischt werden, sollte die Strömung an der Einmischstelle möglichst turbulent sein. Die Einmischstelle ist der Ort, wo das jeweilige Edukt auf die rückgeführte neutralisierte Lösung trifft.

In einer bevorzugten Ausführungsform der vorliegenden Erfindung wird mindestens eines der Edukte in ein, vorzugsweise werden alle Edukte in ein, besonders bevorzugt werden alle Edukte in ein gemeinsames, Venturi-Rohr dosiert.

Ein Venturi-Rohr ist eine in ihrer Länge begrenzte Rohrverengung, in der Druckverlust weitgehend reversibel in kinetische Energie umgewandelt wird. Dazu wird der Querschnitt F₁ auf der Strecke L₁ auf den Querschnitt F₂ vermindert, der Querschnitt F₂ wird auf der Strecke L₂ konstant gehalten und anschließend wird der Querschnitt F₂ auf der Strecke L₃ wieder auf den Querschnitt F₁ geweitet. Dabei ist der Querschnitt F₁ größer als der Querschnitt F₂ und die Länge L₃ größer als die Länge L₁.

Die Dosierung der Edukte für die Neutralisation erfolgt vorzugsweise im Bereich der Strecke L₂ mit dem Querschnitt F₂.

Die optimale Auslegung eines Venturi-Rohrs ist dem Fachmann an sich bekannt. Vorzugsweise wird das Venturi-Rohr so ausgelegt, dass der Druck im Bereich der Strecke L₂ weniger als der Umgebungsdruck beträgt (Saugförderung) und/oder das die Strömung im Bereich der Strecke L₂ turbulent ist, wobei die Reynolds-Zahl mindestens 1000, vorzugsweise mindestens 2000, besonders bevorzugt mindestens 3000, ganz besonders bevorzugt mindestens 4000, und üblicherweise weniger als 10.000.000 betragen sollte.

Das erfindungsgemäße kontinuierliche Neutralisationsverfahren ermöglicht es polymerisationsempfindliche ethylenisch ungesättigte Säuren besonders schonend zu neutralisieren. Lokale Überhitzungen, die eine unerwünschte Polymerisation auslösen können, werden zuverlässig vermieden.

Das erfindungsgemäße kontinuierliche Neutralisationsverfahren ist auch für diskontinuierliche Polymerisationsverfahren geeignet. Beispielsweise kann der Behälter B gemäß Figur 1 als Vorratsbehälter für eine diskontinuierliche Polymerisation verwendet werden. Das kontinuierliche Neutralisationsverfahren ermöglicht gegenüber der konventionellen diskontinuierlichen Neutralisation eine deutlich verbesserte Wärmeabfuhr.

Ein weiterer Gegenstand der vorliegenden Erfindung ist ein Verfahren zur Herstellung wasserabsorbierender Polymere, in dem eine gemäß dem erfindungsgemäßen Neutralisationsverfahren hergestellte neutralisierte Lösung als Monomerlösung verwendet wird.

Vorzugsweise wird das erfindungsgemäße kontinuierliche Neutralisationsverfahren mit einem kontinuierlichen Polymerisationsverfahren kombiniert, wobei vorzugsweise alle Verfahrensschritte, wie Neutralisation, Polymerisieren, Trocknen, Mahlen, Sieben, Nachvernetzen, Sieben, kontinuierlich durchgeführt werden.

Die wasserabsorbierenden Polymere werden beispielsweise durch Polymerisation einer Monomerlösung, enthaltend
a) mindestens eine ethylenisch ungesättigte Carbonsäure,
b) mindestens einen Vernetzer,
c) wahlweise ein oder mehrere mit dem Monomeren a) copolymerisierbare ethylenisch und/oder allylisch ungesättigte Monomere und
d) wahlweise ein oder mehrere wasserlösliche Polymere, auf die die Monomere a), b) und ggf. c) zumindest teilweise aufgepfropft werden können,
erhalten.

Geeignete ethylenisch ungesättigte Carbonsäuren a) sind beispielsweise Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 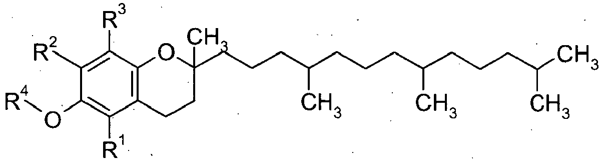 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847, EP-A 0 559 476, EP-A 0 632 068, WO 93/21237, WO 03/104299, WO 03/104300, WO 03/104301 und DE-A 103 31 450 beschrieben, gemischte Acrylate, die neben Acrylatgruppen weitere ethylenisch ungesättigte Gruppen enthalten, wie in DE-A 103 31 456 und WO 04/013064 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylämid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Menge an Vernetzer b) beträgt vorzugsweise 0,01 bis 1 Gew.-%, besonders bevorzugt 0,05 bis 0,5 Gew.-%, ganz besonders bevorzugt 0,1 bis 0,3 Gew.-%, jeweils bezogen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Üblicherweise werden die Monomerlösungen vor der Polymerisation weitgehend von Sauerstoff befreit (Inertisierung), beispielsweise mittels Durchströmen mit einem inerten Gas, vorzugsweise Stickstoff. Dadurch werden die Polymerisationsinhibitoren in ihrer Wirkung deutlich abgeschwächt. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE-A 199 41 423, EP-A 0 686 650, WO 01/45758 und WO 03/104300 beschrieben.

Wasserabsorbierende Polymere werden üblicherweise durch Polymerisation einer wässrigen Monomerlösung und wahlweise einer anschließenden Zerkleinerung des Hydrogels erhalten. Geeignete Herstellverfahren sind in der Literatur beschrieben. Wasserabsorbierende Polymere können beispielsweise erhalten werden durch
- Gelpolymerisation im Batchverfahren bzw. Rohrreaktor und anschließender Zerkleinerung im Fleischwolf, Extruder oder Kneter (EP-A 0 445 619, DE-A 198 46 413)
- Polymerisation im Kneter, wobei durch beispielsweise gegenläufige Rührwellen kontinuierlich zerkleinert wird, (WO 01/38402)
- Polymerisation auf dem Band und anschließende Zerkleinerung im Fleischwolf, Extruder oder Kneter (DE-A 38 25 366, US 6,241,928)
- Emulsionspolymerisation, wobei bereits Perlpolymerisate relativ enger Gelgrö-ßenverteilung anfallen (EP-A 0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO 02/94328, WO 02/94329)

Die Umsetzung wird vorzugsweise in einem Kneter, wie beispielsweise in WO 01/38402 beschrieben, oder auf einem Bandreaktor, wie beispielsweise in EP-A 0 955 086 beschrieben, durchgeführt.

Die Neutralisation kann auch teilweise nach der Polymerisation auf der Stufe des Hydrogels durchgeführt werden. Es ist daher möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso weißer, je höher der Feststoffgehalt des Gels ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 800 µm, und vorzugsweise über 100 µm, besonders bevorzugt über 150 µm, ganz besonders bevorzugt über 200 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C 33 14 019, DE-C 35 23 617 und EP-A 0 450 922 beschrieben, oder ß-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Oberflächennachvernetzer beschrieben.

Die Nachvernetzung wird üblicherweise so durchgeführt, dass eine Lösung des Oberflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

### Beispiele

Die Peaktemperatur der folgenden Beispiele wurde mit Fluent 6.0 berechnet. Die Peaktemperatur ist die höchste im System auftretende Temperatur. Das Programm kann beispielsweise über Fluent Deutschland GmbH, Birkenweg 14a, D-64295 Darmstadt, bezogen werden. Weitere Bezugsquellen sind unter www.fluent.com zu finden.

### Beispiele 1 bis 6

Für die Beispiele 1 bis 6 wurde eine Vorrichtung, wie in Figur 1 beschrieben, angenommen. Für die Berechnung wurde der Durchmesser der Ringleitung R zu 20 cm, der Durchmesser der Zuführungen Z₁ bis Z₃ jeweils zu 5 cm, der Massenstrom in der Ringleitung R vor der Zuführung Z₃ zu 349 t/h, die Temperatur des Massenstromes in der Ringleitung R vor der Zuführung Z₃ zu 26°C, der Abstand zwischen Zuführung Z₃ und Zuführung Z₁ zu 20 cm, der Abstand zwischen Zuführung Z₁ und Zuführung Z₂ zu 20 cm, der Massenstrom Acrylsäure zu 11,5 t/h, der Massenstrom 50gew.-%ige Natronlauge zu 8,4 t/h und der Massenstrom Wasser zu 15 t/h festgelegt.

Die Beispiele 1 bis 3 belegen den Einfluss der Dosierreihenfolge.

In den Beispielen 4 bis 6 werden Natronlauge und Wasser vorgemischt. In den Beispielen 5 und 6 wurde die Berechnung zusätzlich um eine Kühlung des Natronlauge/Wasser-Gemisches vor dem Eintritt in die Ringleitung R auf die angegebene Temperatur ergänzt.

Die Beispiele belegen, wie über die Dosierreihenfolge die Peaktemperatur beeinflusst werden kann.

| Beispiel | Zuführung | | | Temperatur | | | Peaktemperatur |
|---|---|---|---|---|---|---|---|
| | Z₃ | Z₁ | Z₂ | NaOH | H₂O | AS | |
| 1**) | H₂O | AS | NaOH | 23°C | 23°C | 23°C | 64°C |
| 2 | NaOH | H₂O | AS | 23°C | 23°C | 23°C | 47°C |
| 3**) | AS | H₂O | NaOH | 23°C | 23°C | 23°C | 64°C |
| 4 | - | NaOH/H₂O | AS | 23°C | 23°C | 23°C | 43°C |
| 5 | - | NaOH/H₂O | AS | 23°C*) | 23°C*) | 15°C | 38°C |
| 6 | - | NaOH/H₂O | AS | 15°C*) | 15°C*) | 15°C | 35°C |

| | | | | | | | |
|---|---|---|---|---|---|---|---|
| NaOH: 50gew.-%ige Natronlauge H₂O: Wasser AS: Acrylsäure *) Temperatur der NaOH/H₂O-Mischung **) Vergleichsbeispiele | | | | | | | |

### Beispiele 7 bis 9

Die Beispiele 7 bis 9 wurden analog zu den Beispielen 1 bis 6 berechnet.

Für die Berechnung der Beispiele 7 bis 9 wurde angenommen, dass eine NaOH/H₂O-Mischung und Acrylsäure in ein gemeinsames Venturi-Rohr dosiert werden. Die Länge des Venturi-Rohres wurde zu 93,2 cm festgelegt, wobei sich das Venturi-Rohr über eine Strecke von 8,4 cm auf einen Durchmesser von 10 cm verjüngt, über eine Strecke von 27,6 cm den Durchmesser von 10 cm beibehält und sich über eine Strecke von 57 cm wieder auf einen Durchmesser von 20 cm weitet. Der Abstand der Zuführung Z₁ von der Stelle, wo sich das Venturi-Rohr auf 10 cm verjüngt hat, wurde zu 5 cm, der Abstand der Zuführung Z₂ von der Zuführung Z₁ wurde zu 8 cm und der Durchmesser der jeweils zwei Zuführungen Z₁ und Z₂ wurde zu 3,5 cm angenommen. Die jeweils beiden Zuführungen Z₁ bzw. Z₂ sind gegenüber angeordnet, wobei die Verbindungsachse der beiden Zuführungen Z₁ gegenüber der Verbindungsachse der beiden Zuführungen Z₂ um 90° gedreht ist.

In den Beispielen 7 bis 9 wurde zusätzlich der Einfluss des Massenstromes in der Ringleitung R untersucht. In den Beispielen 8 und 9 wurde der Massenstrom in der Ringleitung R daher rechnerisch um 50% bzw. 90% reduziert.

| Beispiel | Mischungsverhältnis | Zuführung | | Temperatur | | Peaktemperatur |
|---|---|---|---|---|---|---|
| | | Z₁ | Z₂ | NaOH/H₂O | AS | |
| 7 | 1 : 10 | NaOH/H₂O | AS | 15°C | 15°C | 27°C |
| 8 | 1 : 5 | NaOH/H₂O | AS | 15°C | 15°C | 46°C |
| 9 | 1 : 1 | NaOH/H₂O | AS | 15°C | 15°C | 69°C |

Die Berechnungen belegen auch die Vorteile des Venturi-Rohres gegenüber der einfachen Dosierung (Vergleich der Beispiele 6 und 7).

## Patentansprüche

1. Neutralisationsverfahren, wobei mindestens eine ethylenisch ungesättigte Carbonsäure zumindest teilweise mit einer Base neutralisiert wird, **dadurch gekennzeichnet, dass** die Neutralisation kontinuierlich durchgeführt wird, die Temperatur der ethylenisch ungesättigten Carbonsäure von 0 bis 40°C beträgt, die neutralisierte Lösung gekühlt und teilweise in die Neutralisation rückgeführt wird, die rückgeführte neutralisierte Lösung in der Neutralisation nacheinander mit einer Base und ethylenisch ungesättigter Carbonsäure versetzt wird und die Temperatur der neutralisierten Lösung weniger als 70°C beträgt.

2. Verfahren gemäß Anspruch 1, **dadurch gekennzeichnet, dass** die Carbonsäure und/oder die Base mit Wasser verdünnt wird.

3. Verfahren gemäß Anspruch 1 oder 2, **dadurch gekennzeichnet, dass** die neutralisierte Lösung auf eine Temperatur von weniger als 50°C gekühlt wird.

4. Verfahren gemäß einem der Ansprüche 1 bis 3, **dadurch gekennzeichnet, dass** zwischen 25 und 99% der neutralisierten Lösung rückgeführt werden.

5. Verfahren gemäß einem der Ansprüche 1 bis 4, **dadurch gekennzeichnet, dass** zwischen 50 und 95% der neutralisierten Lösung rückgeführt werden.

6. Verfahren gemäß einem der Ansprüche 1 bis 5, **dadurch gekennzeichnet, dass** die Base wässriges Alkali ist.

7. Verfahren gemäß einem der Ansprüche 1 bis 6, **dadurch gekennzeichnet, dass** die Temperatur der neutralisierten Lösung weniger als 50°C beträgt.

8. Verfahren gemäß einem der Ansprüche 1 bis 6, **dadurch gekennzeichnet**, die dass rückgeführte neutralisierte Lösung in der Neutralisation nacheinander mit Wasser, einer Base und ethylenisch ungesättigter Carbonsäure versetzt wird.

9. Verfahren gemäß einem der Ansprüche 6 bis 8 **dadurch gekennzeichnet, dass** die ethylenisch ungesättigte Carbonsäure eine Temperatur von 15 bis 25°C und/oder das wässrige Alkali einen Alkaligehalt von weniger als 25 Gew.-% und eine Temperatur von 15 bis 30°C oder das wässrige Alkali einen Alkaligehalt von mindestens 25 Gew.-% und eine Temperatur von 30 bis 50°C und/oder falls eingesetzt das Wasser eine Temperatur von 15 bis 30°C aufweist.

10. Verfahren gemäß einem der Ansprüche 1 bis 9, **dadurch gekennzeichnet, dass** mindestens eine Dosierstelle für Base, ethylenisch ungesättigte Carbonsäure und wahlweise Wasser als Venturi-Rohr ausgebildet ist.

11. Verfahren gemäß einem der Ansprüche 6 bis 10, **dadurch gekennzeichnet, dass** das wässrige Alkali Natronlauge und/oder die ethylenisch ungesättigte Carbonsäure Acrylsäure ist.

12. Verfahren zur Herstellung wasserabsorbierender Polymere, umfassend ein Neutralisationsverfahren gemäß einem der Ansprüche 1 bis 11.

## Claims

1. A neutralization process in which at least one ethylenically unsaturated carboxylic acid is neutralized at least partly with a base, wherein the neutralization is carried out continuously, the temperature of the ethylenically unsaturated carboxylic acid is from 0 to 40°C, the neutralized solution is cooled and partly recycled into the neutralization, the recycled neutralized solution, in the neutralization, is admixed successively with a base and ethylenically unsaturated carboxylic acid and the temperature of the neutralized solution is less than 70°C.

2. The process according to claim 1, wherein the carboxylic acid and/or the base is diluted with water.

3. The process according to claim 1 or 2, wherein the neutralized solution is cooled to a temperature of less than 50°C.

4. The process according to any of claims 1 to 3, wherein between 25 and 99% of the neutralized solution is recycled.

5. The process according to any of claims 1 to 4, wherein between 50 and 95% of the neutralized solution is recycled.

6. The process according to any of claims 1 to 5, wherein the base is aqueous alkali.

7. The process according to any of claims 1 to 6, wherein the temperature of the neutralized solution is less than 50°C.

8. The process according to any of claims 1 to 6, wherein the recycled neutralized solution, in the neutralization, is admixed successively with water, a base and ethylenically unsaturated carboxylic acid.

9. The process according to any of claims 6 to 8, wherein the ethylenically unsaturated carboxylic acid has a temperature of from 15 to 25°C and/or the aqueous alkali has an alkali content of less than 25% by weight and a temperature of from 15 to 30°C or the aqueous alkali has an alkali content of at least 25% by weight and a temperature of from 30 to 50°C and/or, if used, the water has a temperature of from 15 to 30°C.

10. The process according to any of claims 1 to 9, wherein at least one metering point for base, ethylenically unsaturated carboxylic acid and, if desired, water is designed as a Venturi tube.

11. The process according to any of claims 6 to 10, wherein the aqueous alkali is sodium hydroxide solution and/or the ethylenically unsaturated carboxylic acid is acrylic acid.

12. A process for preparing water-absorbing polymers, comprising a neutralization process according to any of claims 1 to 11.

## Revendications

1. Procédé de neutralisation, selon lequel au moins un acide carboxylique éthyléniquement insaturé est neutralisé au moins partiellement avec une base, **caractérisé en ce que** la neutralisation est réalisée en continu, la température de l'acide carboxylique éthyléniquement insaturé est de 0 à 40 °C, la solution neutralisée est refroidie et partiellement recyclée dans la neutralisation, la solution neutralisée recyclée dans la neutralisation est mélangée successivement avec une base et un acide carboxylique éthyléniquement insaturé, et la température de la solution neutralisée est inférieure à 70 °C.

2. Procédé selon la revendication 1, **caractérisé en ce que** l'acide carboxylique et/ou la base sont dilués avec de l'eau.

3. Procédé selon la revendication 1 ou 2, **caractérisé en ce que** la solution neutralisée est refroidie à une température inférieure à 50 °C.

4. Procédé selon l'une quelconque des revendications 1 à 3, **caractérisé en ce qu'**entre 25 et 99 % de la solution neutralisée est recyclée.

5. Procédé selon l'une quelconque des revendications 1 à 4, **caractérisé en ce qu'**entre 50 et 95 % de la solution neutralisée est recyclée.

6. Procédé selon l'une quelconque des revendications 1 à 5, **caractérisé en ce que** la base est un alcali aqueux.

7. Procédé selon l'une quelconque des revendications 1 à 6, **caractérisé en ce que** la température de la solution neutralisée est inférieure à 50 °C.

8. Procédé selon l'une quelconque des revendications 1 à 6, **caractérisé en ce que** la solution neutralisée recyclée dans la neutralisation est mélangée successivement avec de l'eau, une base et l'acide carboxylique éthyléniquement insaturé.

9. Procédé selon l'une quelconque des revendications 6 à 8, **caractérisé en ce que** l'acide carboxylique éthyléniquement insaturé présente une température de 15 à 25 °C et/ou l'alcali aqueux présente une teneur en alcali inférieure à 25 % en poids et une température de 15 à 30 °C ou l'alcali aqueux présente une teneur en alcali d'au moins 25 % en poids et une température de 30 à 50 °C et/ou, si elle est utilisée, l'eau présente une température de 15 à 30 °C.

10. Procédé selon l'une quelconque des revendications 1 à 9, **caractérisé en ce qu'**au moins un emplacement d'alimentation pour la base, l'acide carboxylique éthyléniquement insaturé et éventuellement l'eau est configuré sous la forme d'un tube Venturi.

11. Procédé selon l'une quelconque des revendications 6 à 10, **caractérisé en ce que** l'alcali aqueux est la soude caustique et/ou l'acide carboxylique éthyléniquement insaturé est l'acide acrylique.

12. Procédé de fabrication de polymères absorbant l'eau, comprenant un procédé de neutralisation selon l'une quelconque des revendications 1 à 11.
